(19) Europäisches Patentamt

European Patent Office

Office européen des brevets

(11) Veröffentlichungsnummer: **0 091 577**
**B1**

# (12) EUROPÄISCHE PATENTSCHRIFT

(45) Veröffentlichungstag der Patentschrift:
28.10.87

(21) Anmeldenummer: 83102827.9

(22) Anmeldetag: 22.03.83

(51) Int. Cl.⁴: **G 01 V 3/12, A 61 B 5/06**

(54) Vorrichtung zum Ermitteln der Position einer Sonde.

(30) Priorität: 25.03.82 DE 3211003

(43) Veröffentlichungstag der Anmeldung:
19.10.83 Patentblatt 83/42

(45) Bekanntmachung des Hinweises auf die Patenterteilung:
28.10.87 Patentblatt 87/44

(84) Benannte Vertragsstaaten:
AT CH DE FR GB IT LI NL

(56) Entgegenhaltungen:
US - A - 3 466 742
US - A - 3 653 050
US - A - 4 096 862
US - A - 4 176 662

NATURE, Band 179, Nr. 4572, 15. Juni 1957, Seiten 1239-1240, Basingstoke, G.B. R. STUART MACKAY et al.: "Endoradiosonde"
CONTROL ENGINEERING, Band 8, Nr. 12, Dezember 1961, Seite 119, USA B. JACOBSEN: "Servoed antenna tracks radio "Pill""

(73) Patentinhaber: **Kunke, Stefan, Dr.med.,
Max-Planck-Strasse 60, D-6500 Mainz-Gonsenheim (DE)**

(72) Erfinder: **Kunke, Stefan, Dr.med.,
Max-Planck-Strasse 60, D-6500 Mainz-Gonsenheim (DE)**

(74) Vertreter: **Kossobutzki, Walter, Dipl.-Ing., Waldstrasse 6,
D-5419 Helferskirchen (DE)**

**Beschreibung**

Die Erfindung bezieht sich auf eine Vorrichtung zum Ermitteln der jeweiligen Position des vorderen Endes einer in einen lebenden Körper eingeführten Sonde, bestehend aus einem ausserhalb des Körpers bewegbaren, elektromagnetische Wellen erzeugenden Sender und einen an der Sonde angebrachten Empfänger mit einem darin enthaltenen Sensor, welcher auf die Feldstärke der auf den Empfänger auftreffenden Wellen des Senders anspricht und dessen optische und/oder akustische Anzeige eine Funktion der jeweiligen Feldstärke am Empfänger ist.

Aus der US-Patentschrift 4 176 662 ist eine Vorrichtung zur Lokalisierung einer Coloskopie-Sonde (Dickdarmspiegelung) bekannt, die eine Dicke bzw. einen Durchmesser von etwa 2,0 cm aufweist. Die Sonde besitzt an ihrer Spitze bzw. in ihrem Verlauf einen oder mehrere als Wandler ausgebildete Sender, der bzw. die elektromagnetische Wellen aussendet bzw. aussenden, die von ausserhalb des menschlichen Körpers befindlichen Empfängern empfangen werden. Die Zuleitungen zu derartigen Sendern müssen eine flexible Abschirmung besitzen. Zusätzlich ist ein hoher technischer Aufwand erforderlich, um eine lokalisierte Abstrahlung von den jeweiligen Sender-Punkten der Sonde zu erreichen. Neben dem hohen materiellen Aufwand muss die Sonde einen ausreichenden Platz für den Sender bieten, das bei dem vorhandenen Durchmesser der Sonde ohne weiteres möglich ist. Bei Sonden, die als Venenkatheter verwendet werden und meist einen Durchmesser von unter 2,0 mm aufweisen, ist ein derartiger Sender nicht einsetzbar. Darüber hinaus ist bei einer solchen Vorrichtung, bei der die Sonde selbst zum Sender wird, ein nicht unbeachtliches Risiko von elektrisch bedingten Herzrythmusstörungen vorhanden. Ferner ist es aus dieser Patentschrift bekannt, die Sonde mit einem Resonator auszurüsten, der von einem ausserhalb des Körpers bewegbaren Hochfrequenzsender angeregt wird. Auch eine solche Vorrichtung erfordert einen verhältnismässig grossen Durchmesser der Sonde und ist für Venenkatheter ungeeignet.

Aus der Zeitschrift «Nature», Bd. 179, Nr. 4572 vom 15. 06. 1957, Seiten 1239 und 1240, ist ein Verfahren zur Ortung eines Senders bekannt, der als Miniatursender ausgebildet und so klein ist, dass er von Menschen geschluckt werden kann. Dabei kann der Sender als passiver Telemetriesender in Gestalt eines Resonanzkreises ausgebildet sein, dessen Frequenz mittels eines Griddipmeters von ausserhalb des Körpers beobachtet wird. Ferner ist hier erwähnt, dass die für die Signaldurchdringung als günstig angesehene Frequenz im 100-kHz-Bereich liegt und dass man eine Sonde mit einer kleinen Antenne verfolgen kann.

In der US-Patentschrift 3 466 742 ist es darüber hinaus offenbart, in eine Arterie einen Thermistor-Strömungsmesser einzusetzen, an den ein elektrisches Kabel angeschlossen ist.

Gegenüber dem aus der US-Patentschrift 4 176 662 bekannten Stand der Technik liegt der Erfindung die Aufgabe zugrunde, eine Vorrichtung zum Ermitteln der jeweiligen Position des vorderen Endes einer in einen lebenden Körper eingeführten Sonde zu schaffen, die auch bei Sonden mit kleinem Durchmesser, beispielsweise bei Venenkathetern, eingesetzt werden kann und die jegliche Herzrythmusstörungen ausschliesst.

Zur Lösung dieser Aufgabe ist die Vorrichtung gemäss dem Kennzeichen des Anspruches 1 ausgebildet. Diese Ausbildung gestattet es, die Vorrichtung auch bei kleinen Sonden, insbesondere bei Venenkáthetern, einzusetzen und schliesst das Risiko von elektrisch bedingten Herzrythmusstörungen aus.

Weiter Fortbildungen und Ausgestaltungen der Erfindung sind in den abhängigen Patentansprüchen gekennzeichnet und werden nachstehend in Verbindung mit den Ausführungsbeispiele darstellenden, teilweise schematisch vereinfachten Figuren beschrieben. In diesen sind einander entsprechende Teile mit gleichen Bezugszeichen versehen, und es sind alle zum Verständnis der Erfindung nicht notwendigen Einzelheiten fortgelassen worden.

Es zeigt:

Fig. 1 das Prinzip der Erfindung, wobei eine mit dem Empfangsgerät verbundene Sonde in den menschlichen Körper eingeführt wird und ihre Position durch einen mit dem Sender verbundenen Abstrahlkopf ermittelt wird, wobei der Patient schräg von oben gesehen dargestellt ist;

Fig. 2 in Ergänzung zu Fig. 1 den Abtastvorgang zur Ermittlung der Spitze der Sonde, wobei der Körper des Patienten in Draufsicht dargestellt ist;

Fig. 3 ein Diagramm, welches den Verlauf der Feldstärke entlang der abgetasteten Oberfläche des Körpers zeigt;

Fig. 4 ein Diagramm, welches einen anderen Verlauf der am Empfänger gemessenen Feldstärke bei Bewegung zeigt, wenn der Abstrahler über dem Körper bewegt wird;

Fig. 5 die Ausführungsform einer als Vena-Cava-Katheter ausgebildeten Sonde mit zwei im vorderen Bereich der Sonde verbundenen Leiter, in isometrischer Darstellung;

Fig. 6 eine andere, zwei Leiterpaare enthaltende, Ausführungsform der Sonde, im Querscnitt;

Fig. 7 einen Teil einer anderen Ausführungsform der in Fig. 3 dargestellten Sonde, jedoch mit in Längsrichtung verdrillten Leitern;

Fig. 8 eine Variante der Ausführungsform nach Fig. 7, bei welcher der in die Sonde eingebettete Leiter am vorderen Ende der Sonde als Spule gewickelt ist und eine Zuführung im wesentlichen in Längsrichtung der Sonde verlaufend, und die andere Zuleitung schraubenförmig ausgebildet ist;

Fig. 9 die Topographie der grösseren venösen Gefässe im Thoraxraum mit Lagebeziehung zum Herzen und mögliche Positionen eingeführter Sonden bzw. Katheter;

Fig. 10 eine weitere Variante der in Fig. 5 dargestellten Ausführungsform mit einer zweiteiligen

Sonde, deren äusserer Teil als Schlauch ausgebildet ist und deren innerer, herausziehbar gelagerter Teil die Leiter enthält;

Fig. 11 als technisches Detail eine vorteilhafte Anordnung der Leiter im inneren Teil der in Fig. 10 dargestellten Sonde.

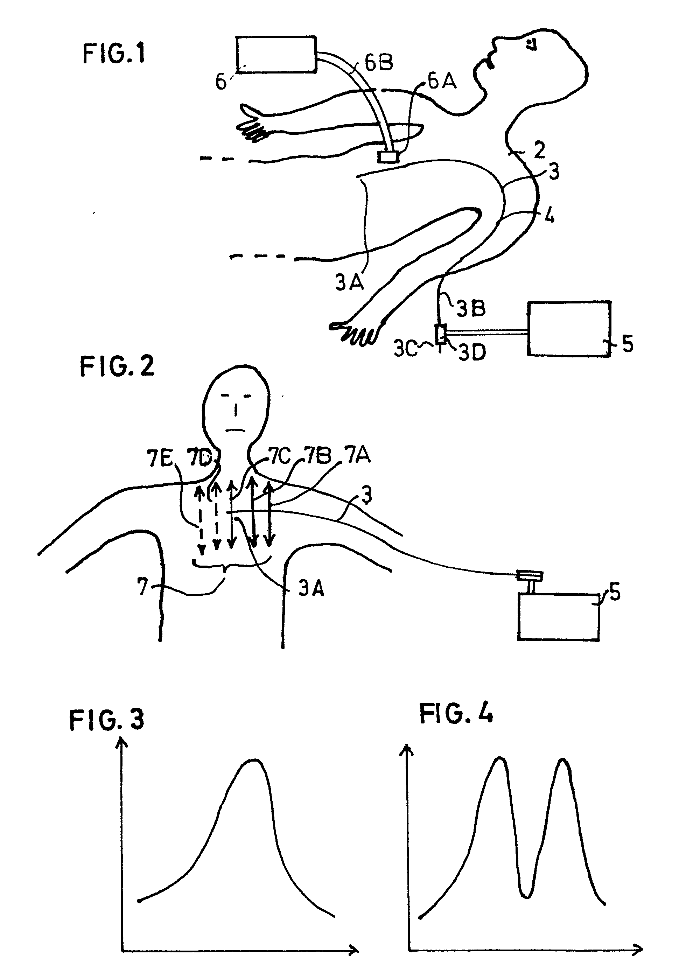

In Figur 1 und 2 ist in stark vereinfachter Weise dargestellt, wie die Lage der in den Körper 2 des Patienten eingeführten Sonde 3 von aussen bestimmt wird. Die Sonde 3 enthält einen metallischen Leiter 4, der vom vorderen Ende 3A der Sonde 3 sich bis zum ausserhalb des Körpers befindlichen Bereich 3B erstreckt. An dem äusseren Ende 3C der Sonde befindet sich eine Anschlussvorrichtung 3D, welche dazu dient, im Bedarfsfall Injektionen oder Infusionsflüssigkeit in die Sonde einzuführen bzw. Blutproben zu entnehmen oder um den Druckverlauf am vorderen Ende der Sonde zu registrieren. In der Anschlussvorrichtung 3D ist ferner ein Anschluss an den in der Sonde befindlichen Leiter 4 vorgesehen, durch welchen dieser Leiter mit dem Eingang des Empfängers 5 verbunden wird.

Es ist in vielen Fällen vorteilhaft, insbesondere falls die Messung durch andere Wellen gestört wird, die beispielsweise von elektrotherapeutischen Geräten oder anderen Störquellen ausgestrahlt werden, wenn der Sender Wellen verschiedener Frequenz abstrahlen kann. Der Empfänger muss entweder einen breiten Frequenzbereich empfangen können oder aber durch eine Umschalteinrichtung auf die jeweils vorgesehene Frequenz des Senders umschaltbar sein. Der Leiter 4 befindet sich in der Wandung der Sonde 3; er ist in den Figuren 1 und 2 der besseren Übersichtlichkeit der Darstellung nicht durch eine zusätzliche Linie dargestellt und sein Verlauf ist bei dem gewählten Zeichenmassstab praktisch mit der Linie der Sonde 3 identisch.

Der Sender 6 speist eine Vorrichtung 6A zur Abstrahlung der erzeugten elektromagnetischen Wellen. Diese ist über ein biegsames Kabel 6B mit dem Sender verbunden. Versuche haben gezeigt, dass ein Löschkopf der bei Tonbandgeräten verwendeten Bauart sich für die Abstrahlung der erzeugten elektromagnetischen Wellen eignet. Der Sender kann jedoch ganz klein ausgeführt sein und die Vorrichtung zur Abstrahlung der erzeugten Wellen in den Sender integriert.

Diese Ausführungsform ist insbesondere für Anwendung bei Notfällen geeignet.

Um nun den Verlauf der in den Körper eingeführten Sonde, insbesondere die Stellen des vorderen Endes 3A zu ermitteln, wird der die Wellen abstrahlende Teil des Senders oder – bei integrierter Ausführung – der Sender selbst quer zur vermuteten Sondenrichtung über die Oberfläche des Körpers bewegt, wie dies durch die in Fig. 2 gezeichneten Pfeile 7 angedeutet ist. Sobald die vom Sender ausgestrahlten Wellen in dem als Antenne wirkenden Leiter 4 ein Signal erzeugen, wird dies am Empfänger 5 optisch, z.B. mittels eines Messinstrumentes oder akustisch durch einen kleinen Lautsprecher angezeigt. Bei Bewegung des Strahles entlang der Pfeile 7A, 7B, 7C wird der Empfänger ein Signal geben. Bei Bewegung entlang der Pfeile 7D und 7E wird der Empfänger entweder nur ein sehr schwaches oder gar kein Signal geben. Daraus ist sofort erkennbar, dass das vordere Ende 3A der Sonde im Bereich sich zwischen der durch die Pfeile 7C und 7D in Fig. 2 gekennzeichneten Abtastzone befindet. Bei maximaler Annäherung des Strahles an die Sonde weist auch das Signal am Empfänger einen Extremwert auf. Dieser kann entweder ein Maximum sein, wie in Fig. 3 dargestellt, oder er kann auch – je nach spezieller Ausbildung des Strahlers und Anordnung der Leiter 4 in der Sonde – ein minimaler Extremwert sein, wie dies in Fig. 4 dargestellt ist. In diesen beiden Diagrammen ist als Abszisse die Längsbewegung entlang der Körperoberfläche, als Ordinate die jeweilige Empfangsfeldstärke dargestellt.

Nach Auffinden des Ortes, an dem sich das vordere Ende oder ein anderer Teil der Sonde befindet, kann auch die jeweilige Richtung der Sonde an der gemessenen Stelle ermittelt werden. Zu diesem Zwecke wird der Strahler entweder um eine im wesentlichen senkrecht zur Körperoberfläche verlaufende Achse gedreht und/ oder er wird gekippt, damit festgestellt werden kann, bei welcher Einstellung des Strahlers das Empfangssignal den Maximalwert erreicht. Wird der jeweilige Verlauf der Sonde auf der Haut des Patienten markiert, dann kann dies eine wertvolle Hilfe für den Arzt beim Einführen des Katheters sein.

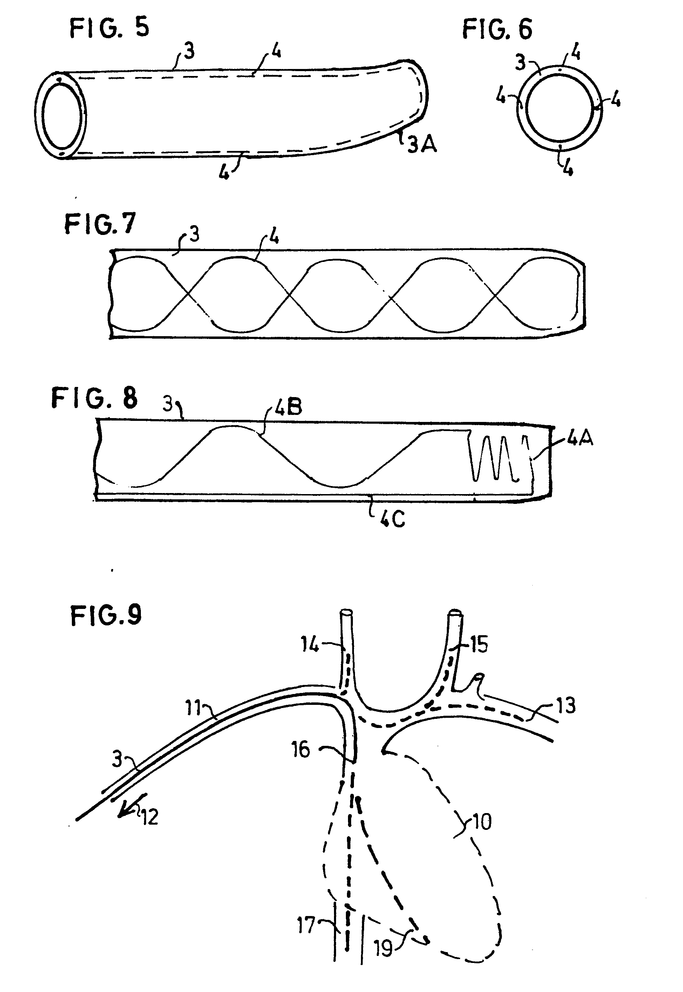

In Fig. 5 ist das vordere Ende 3A einer Sonde und der Verlauf der darin eingebetteten Leiter 4 dargestellt. Bei dieser Ausführungsform der Sonde verlaufen die beiden Leiter im wesentlichen parallel, im Inneren der Wandung der Sonde sind sie nahe dem vorderen Ende im Bereich 4A miteinander verbunden, so dass der eine Leiter als Hinleitung und der andere als Rückleitung aufgefasst werden kann.

Diese Ausführungsform der Sonde eignet sich – wie Versuche ergeben haben – nicht nur gut zur Ermittlung der Lage am Messort, sondern auch zur Feststellung, ob und inwieweit die Sonde am Messort um ihre Längsrichtung verdreht ist. Dies ist insbesondere bei solchen Sonden nützlich, welche im Bereich ihrer Spitze eine leicht bogenförmige Krümmung aufweisen; diese Krümmung dient dazu, um bei Verdrehen der Sonde um ihre Längsachse das Hineinschlüpfen der Sonde in einen bestimmten Zweig einer Gefässverzweigung zu erleichtern.

Die Lage der einzelnen Leiter der im Körper befindlichen Sonde kann durch eine gegebenenfalls unbeabsichtigte schraubenförmige Verdrehung der Sonde in verschiedenen Bereichen der Sonde relativ zur Richtung der elektromagnetischen Wellen im Messfeld verschieden sein. Es kann daher günstig sein, mehrere in Längsrichtung der Sonde verlaufende Leiter anzuordnen und das Messgerät jeweils an zwei diametral gegenüberliegenden Leiter anzuschliessen. Hierdurch kann die durch Lokalisierung jeweils günstigste Lage der Leiter bei der Messung verwendet werden.

Fig. 6 zeigt den Querschnitt durch eine derartige Sonde, bei der in Längsrichtung vier Leiter 4 verlaufen; jeweils zwei diametral gegenüberliegende Leiter sind im Bereich des vorderen Endes der Sonde miteinander verbunden.

Fig. 7 zeigt in Seitenansicht eine Sonde 3, bei der zwei diametral gegenüberliegende Leiter 4 gegeneinander in Längsrichtung der Sonde verdrillt sind. Die Sonde besteht üblicherweise aus transparentem Material, so dass der durchscheinende Leiter gut erkennbar ist. Diese Ausführungsform mit verdrillten Leitern ist günstig, wenn die Lage der Sonde schnell festgestellt werden soll. Es ist nämlich möglich, dass bei einer Sonde mit zwei Leitern diese beiden im Messfeld eine derartige Lage haben, dass die aufgenommenen Signale einander entgegenwirken und daher im Empfänger nur ein sehr geringer Ausschlag angezeigt wird. Man kann nun die Sonde um ihre Längsachse verdrehen und dadurch einen wesentlich höheren, schärfer ausgeprägten Maximalwert bei der Messung erzielen. Doch erfordert dies etwas Zeit und durch Verwendung einer Sonde mit Leiterführung gemäss Fig. 7 kann dieser Zeitaufwand eingespart werden.

Fig. 8 zeigt eine weitere Ausführungsform einer Sonde (aus transparentem Material), an derem vorderen Ende der Leiter zu einer Spule A4 gewikkelt ist. Ein Ende dieser Spule ist an einen in Längsrichtung der Sonde 3 gewendelten Leiter 4B, das andere Ende der Spule an einen im wesentlichen parallel zur Längsrichtung der Sonde verlaufenden Leiter 4C angeschlossen. Bei Verwendung dieser Sonden spricht deren vorderes Ende besonders deutlich auf die von aussen zugeführten Wellen an und ist somit besonders leicht und deutlich von dem übrigen Bereich der Sonde zu unterscheiden.

In Fig. 9 ist die Topographie der grösseren venösen Gefässe im Thoraxraum in der Nähe des Herzens 10 dargestellt. 11 bezeichnet die vena subclavia, die in Richtung des Pfeiles 12 in die rechte Vene übergeht, 13 die vena subclavia der gegenüberliegenden Seiten, 14 und 15 die rechte bzw. linke grosse Halsvene und 16 die obere Hohlvene und 17 die untere Hohlvene.

In Fig. 9 ist angedeutet, welche Wege eine entgegen der Richtung des Pfeiles 12 durch die rechte Armvene eingeführte Sonde 3 einschlagen kann. Wenn eine Infusion für längere Zeit zugeführt werden soll, dann ist es wünschenswert, sie in einen kräftigen Blutstrom einzuleiten, und zwar wird hierfür bevorzugt die Hohlvene 16 gewählt. Handelt es sich hingegen um die Einführung der Sonde für einen Herzschrittmacher, dann ist es wünschenswert, dass diese in den Bereich der gestrichelten Linie 19 in die rechte Herzkammer eingeführt wird.

Beim Einführen der Sonde durch die vena subclavia 11 kann es vorkommen, dass das vordere Ende der Sonde nicht in der gewünschten Richtung vorgeschoben wird, sondern entweder in die rechte oder linke grössere Halsvene 14 bzw. 15, in die vena subclavia 13 oder die untere Hohlvene 17 gelangt. Es kann aber auch der Fall sein, dass die Spitze der Sonde an einer Gefässwand hängenbleibt und der anschliessende Teil der Sonde sich beim weiteren Vorschieben aufrollt. Wird die Lage des vorderen Endes der Sonde hierbei nicht genau kontrolliert, dann können bei Betrachtung der Länge des eingeführten Teiles der Sonde Fehlschätzungen über die tatsächliche Lage des vorderen Endes der Sonde entstehen.

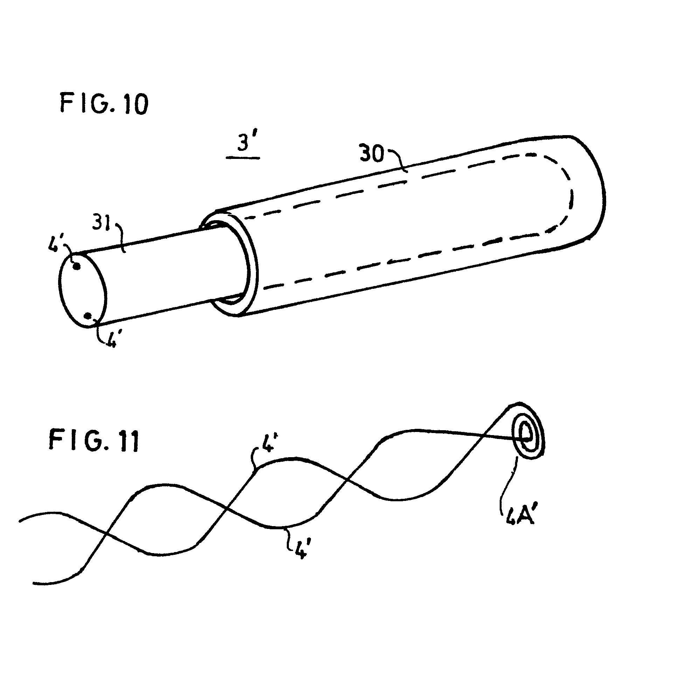

Die in Figur 10 dargestellte Sonde 3' ist zweiteilig ausgeführt und besteht aus einem äusseren als Schlauch ausgebildeten Teil 30 und einem in diesem Schlauch befindlichem Teil 31. Der innere Teil 31 enthält zwei in Längsrichtung miteinander verdrillte Leiter 4'. Diese Leiter 4' sind gegeneinander isoliert und leiten die empfangenen Signale zu dem in Figur 1 dargestellten Empfänger. Infolge der verdrillten Anordnung der Leiter 4' wirkt fast nur der vorderste Teil der dort miteinander verbundenen Leiter als Antenne und dies erleichtert die Lokalisierung des jeweils vordersten Teils der Sonde. Dies ist insbesondere vorteilhaft, wenn die Sonde in den stark gekrümmten Bereich eines Blutgefässes hineingeschoben wird.

Wenn beim Einschieben der Sonde deren vorderer Bereich den vorgesehenen Ort erreicht, kann der innere Teil 31 aus dem schlauchförmigen Teil 30 herausgezogen werden, und es kann durch die Sonde eine Infusionsflüssigkeit eingeführt oder eine Druckmessung vorgenommen werden.

Die Lokalisierung der Spitze der Sonde im Verlauf des Einführens kann ferner noch dadurch erleichtert werden, dass die Leiter im vorderen Teil eine Spule 4A' bilden und die Zuleitungen zu dieser Spule in Längsrichtung verdrillt sind.

Figur 11 zeigt schematisch vereinfacht die Anordnung der Leiter und der Spule 4A' im inneren Teil 31.

Durch die zweiteilige Ausführung der Sonde kann die Herstellung wesentlich vereinfacht werden. Denn es steht dann für die isolierte Anordnung der Leiter genügend Raum zur Verfügung.

Weiterhin ergibt sich der ganz besondere Vorteil, dass in einen handelsüblichen schlauchförmigen Katheter vor dem Einführen ein die Leiter enthaltender innerer Teil eingelegt wird, mit dem dann der Verlauf des Einführens der Sonde genau kontrolliert werden kann.

Bei den vorstehend beschriebenen Ausführungsformen dienen eine oder mehrere in der Sonde befindliche Leiter als Empfangsantenne für den Empfänger und der Sender bzw. der die Wellen abstrahlende Teil wird über die Oberfläche des Körpers bewegt. Bei Einführung einer Sonde in das Herz oder in die Nähe des Herzens ist es günstig, die Leiter der Sonde als Empfangsantenne zu verwenden, weil dann die Belastung des Körpers, insbesondere des Herzens, durch elektrische Ströme und/oder Impulse gering ist.

Die erfindungsgemässe Vorrichtung eignet sich auch dazu, um die Einführung einer Sonde durch die rechte Herzkammer hindurch in die Lungenarterie zu kontrollieren, beispielsweise, wenn der Pulmonaldruck bzw. der Pulmonalkapillardruck gemessen werden soll.

Die Erfindung ist ferner auch anwendbar bei der Einführung von Sonden in das arterielle System, beispielsweise zur Herzkatheteruntersuchung.

In analoger Weise eignet sich die Erfindung auch zur Feststellung der Lage bzw. Kontrolle der Einführung von Sonden in den Magen-Darm-Trakt.

Statt Sonden mit ausgeprägten metallischen in die Wand eingelegten Leitern zu verwenden, können gegebenenfalls auch Sonden verwendet werden, bei denen der schlauchförmige Teil der Sonde auch andere leitfähige Bahnen enthält.

Die erfindungsgemässe Vorrichtung wird mit besonderem Vorteil bei der Einführung von Infusions- oder Herzkathetern verwendet. In beiden Fällen soll die Sonde über die obere Hohlvene bis in unmittelbare Nähe des Herzens oder sogar in das Herz selbst eingeführt werden. Die Sonde kann aber bei der Erfindung über die Armvene unter Umständen nicht den vorgesehenen Weg einschlagen, sondern einen der nachfolgend aufgeführten unerwünschten Weg einschlagen:

1. nach oben in die Halsvene,
2. in die vena subclavia der gegenüberliegenden Seite,
3. zu tief im rechten Herzen liegen (oder nicht weit genug, je nach Anwendungszweck),
4. an dem Herzen vorbeirutschen und in die untere Hohlvene gelangen,
5. sich aufrollen (im Gefässsystem) und so trotz scheinbar grosser Vorschublänge ihren Bestimmungsort nicht erreichen.

Ähnlich verhält es sich bei arteriellen Kathetern und Herzschrittmachersonden, insbesondere wenn diese notfallmässig gelegt werden müssen.

Bei der Anwendung der erfindungsgemässen Vorrichtung wird schnell erkannt, ob das vordere Ende der Sonde einen unerwünschten Weg einschlägt und es können dann gleich entsprechende Massnahmen getroffen werden, damit die Sonde den vorgesehenen Weg einschlägt.

In manchen Fällen, insbesondere für die Herstellung der Katheter, ist es vorteilhaft, wenn die elektrisch leitenden Bahnen, gegebenenfalls auch die Spule, aus in oder auf der Katheterwand fein dispers verteilten leitenden Materialteilchen (z.B. Silberpartikeln) oder anderen elektrisch leitenden Materialien bestehen.

**Patentansprüche**

1. Vorrichtung zum Ermitteln der jeweiligen Position des vorderen Endes (3A) einer in einen lebenden Körper eingeführten Sonde (3), bestehend aus einem ausserhalb des Körpers bewegbaren, elektromagnetischen Wellen erzeugenden Sender (6) und einem an der Sonde angebrachten Empfänger (5), welcher auf die Feldstärke der auf den Empfänger auftreffenden Wellen des Senders anspricht und dessen optische und/oder akustische Anzeige eine Funktion der jeweiligen Feldstärke am Empfänger (5) ist, dadurch gekennzeichnet, dass der Empfänger (5) mit einem metallischen Leiter (4) verbunden ist, welcher sich von dem ausserhalb des Körpers (2) befindlichen Bereich der Sonde (3) bis zu dem vorderen Ende (3A) dieser Sonde erstreckt.

2. Vorrichtung nach Anspruch 1, gekennzeichnet durch eine derartige konstruktive Ausbildung der Sonde (3) bzw. der in ihr enthaltenen Empfangsantenne, dass diese in einem Bereich eine wesentlich höhere Empfindlichkeit für die auf sie auftreffenden elektromagnetischen Wellen hat als im übrigen Bereich.

3. Vorrichtung nach Anspruch 1, dadurch gekennzeichnet, dass die Sonde (3) in ihrem vordersten Bereich eine wesentlich höhere Empfindlichkeit für die auf sie auftreffenden elektromagnetischen Wellen hat als im übrigen Bereich.

4. Vorrichtung nach Anspruch 1 oder 2, dadurch gekennzeichnet, dass in dem Bereich der Sonde (3) mit der höheren Empfindlichkeit eine Spule angeordnet ist, die an zwei Leiter angeschlossen ist.

5. Vorrichtung nach Anspruch 1, dadurch gekennzeichnet, dass die Sonde (3) zwei voneinander isolierte elektrische Leiter (4) und zusätzlich einen dritten elektrischen Leiter enthält, dessen vorderer Endbereich mit dem vorderen Endbereich eines der beiden anderen Leiter verbunden ist.

6. Vorrichtung nach Anspruch 1, dadurch gekennzeichnet, dass die Sonde (3) vier im wesentlichen gleichförmig entlang des Umfanges verteilte Leiter (4) aufweist, von denen jeweils zwei einander diametral gegenüberliegen und in ihren vorderen Endbereichen miteinander verbunden sind.

7. Vorrichtung nach Anspruch 6, dadurch gekennzeichnet, dass eine Umschalteinrichtung zum wahlweisen Anschluss des Empfängers an eines der beiden in ihren vorderen Endbereichen miteinander verbundenen Leiterpaare vorgesehen ist.

8. Vorrichtung nach Anspruch 1, dadurch gekennzeichnet, dass die Sonde (3) zwei in ihrer Längsrichtung schraubenförmig verlaufende, jeweils diametral gegenüberliegende Leiter (4) hat, die in ihrem vorderen Endbereich miteinander verbunden sind.

9. Vorrichtung nach Anspruch 1, dadurch gekennzeichnet, dass die Sonde (3) in ihrem vorderen Endbereich eine an die beiden Leiter (4) angeschlossene Spule (4A) enthält.

10. Vorrichtung nach Anspruch 1, dadurch gekennzeichnet, dass die Sonde (3) in ihrem vorderen Endbereich einen temperaturabhängigen Widerstand, vorzugsweise einen sogenannten Thermistor, und/oder einen physico-chemischen zweiten Sensor, z.B. zur $O_2$-Partialdruckmessung enthält, und dass die Zuleitungen zu diesem temperaturabhängigen Widerstand bzw. physico-chemischen Sensor als Antenne ausgebildet und an den Empfänger (5) angeschlossen sind.

11. Vorrichtung nach Anspruch 1, dadurch gekennzeichnet, dass die Frequenz der vom Sender (6) erzeugten elektromagnetischen Wellen zwischen 15 und 200 kHz beträgt.

12. Vorrichtung nach Anspruch 1, dadurch gekennzeichnet, dass die Stärke und/oder die Frequenz der abgestrahlten elektromagnetischen Wellen wahlweise einschaltbar bzw. umschaltbar sind.

13. Vorrichtung nach Anspruch 1, dadurch gekennzeichnet, dass der Empfänger (5) einen hörbaren Ton erzeugt, dessen jeweilige Frequenz und/oder Lautstärke eine Funktion der empfangenen Feldstärke ist.

14. Vorrichtung nach Anspruch 1, dadurch gekennzeichnet, dass der Empfänger (5) eine optische Anzeigevorrichtung aufweist, welche die Intensität der jeweils gemessenen Feldstärke visuell anzeigt.

15. Vorrichtung nach Anspruch 1, gekennzeichnet durch einen am Sender (6) angeordneten, zweckmässigerweise als Filzstift ausgebildeten Schreibstift zum Zwecke der einfacheren und schnelleren Markierung der ermittelten Position der Sonde (3).

16. Vorrichtung nach Anspruch 14, gekennzeichnet durch eine Hilfseinrichtung, mit der der Schreibstift zum Zwecke der Markierung der Lage bzw. des Verlaufs der Sonde wahlweise in Richtung zum Körper verschiebbar ist.

17. Vorrichtung nach Anspruch 1, dadurch gekennzeichnet, dass der Sender (6) zwei Vorrichtungen zur Erzeugung elektromagnetischer Wellen enthält, die derart angeordnet und bemessen sind, dass die von den beiden Vorrichtungen abgestrahlten Wellen im Messfeld einen Winkel von vorzugsweise weniger als 45° miteinander einschliessen.

18. Vorrichtung nach einem der Ansprüche 1–17, dadurch gekennzeichnet, dass die Sonde aus zwei konzentrisch ineinander angeordneten Teilen (30, 31) besteht, deren äusserer Teil (30) als Schlauch ausgebildet ist.

19. Vorrichtung nach Anspruch 18, dadurch gekennzeichnet, dass der innere Teil (31) in dem äusseren Teil (30) herausziehbar gelagert ist und zwei in Längsrichtung der Sonde sich erstreckende, einerseits mit dem Eingang des Empfängers (5) verbundene und andererseits am vorderen Ende miteinander leitend verbundene Leiter (4′) enthält.

20. Vorrichtung nach Anspruch 18, dadurch gekennzeichnet, dass der innere Teil (31) aus zwei in Längsrichtung der Sonde (3) sich erstreckenden, am vorderen Endbereich miteinander leitend verbundenen Leitern (4′) besteht, die gegeneinander isoliert und in Längsrichtung miteinander verdrillt sind.

21. Vorrichtung nach Anspruch 19 oder 20, dadurch gekennzeichnet, dass am vorderen Ende des inneren Teiles (31) eine wenigstens zwei Windungen enthaltende Spule (4A′) vorgesehen ist, deren beide Enden an je einem der im inneren Teil (31) befindlichen, vorzugsweise untereinander verdrillten Leitern (4′) angeordnet sind.

**Claims**

1. Apparatus for the determination of the respective position of the front end of a probe inserted into a living body, comprising a transmitter, which is movable outside the body and is producing electromagnetic waves, and a receiver attached to the probe containing a sensor which reacts on the field intensity of the waves of the transmitter received by the receiver, and of which the optical and/or acoustical indication is a function of the respective field intensity at the receiver, characterized in that the receiver (5) is connected to a filamentary receiving antenna which extends from the part of the probe (3) being outside the body (2) to the front end (3A) of this probe.

2. Apparatus according to claim 1, characterized by such a constructional configuration of the probe (3) respectively of the receiving antenna contained therein, that this shows in one portion an essentially higher sensibility for the electromagnetic waves striking on it than in the residuary portion.

3. Apparatus according to claim 1, characterized in that the probe (3) comprises in its front portion an essentially higher sensibility for the electromagnetic waves striking on it than in the residuary portion.

4. Apparatus according to claim 1 or 2, characterized in that a coil connected with two conductors is arranged in the portion of the probe (3) with the higher sensibility.

5. Apparatus according to claim 1, characterized in that the probe (3) contains two electrical conductors (4) insulated of each other and in addition a third electrical conductor, of which the front end portion is connected with the front end portion of one of the two other conductors.

6. Apparatus according to claim 1, characterized in that the probe (3) shows four conductors (4) essentially uniformly distributed along the perimeter, of which respectively two conductors lies opposite diametrically and are connected with each other in their front end portion.

7. Apparatus according to claim 6, characterized in that a change-over switching device is provided for an optional connection of the receiver with one of the both pairs of conductors connected with each other in the front end portion.

8. Apparatus according to claim 1, characterized in that the probe (3) comprises two conductors (4), which extend screw-shaped in their longitudinal direction, lie diametrically opposite to each other and are connected with each other in their front end portion.

9. Apparatus according to claim 1, characterized in that the probe (3) contains in its front end portion a coil (4A) connected with the both conductors (4).

10. Apparatus according to claim 1, characterized in that the probe (3) contains in its front end portion a temperature dependent resistor, preferably a so-called thermistor, and/or a second physico-chemical sensor, e.g. for the partial pressure measurement of $O_2$, and that the leads of this temperature dependent resistor respectively of the physico-chemical sensor are formed as an antenna and are connected to a receiver (5).

11. Apparatus according to claim 1, characterized in that the frequency of the electromagnetic waves produced by the transmitter (6) lies between 15 and 200 kHz.

12. Apparatus according to claim 1, characterized in that the intensity and/or the frequency of the radiated electromagnetic waves can optionally be switched on respectively over.

13. Apparatus according to claim 1, characterized in that the receiver (5) produces an audible sound, of which the respective frequency and/or volume is a function of the field intensity.

14. Apparatus according to claim 1, characterized in that the receiver (5) comprises an optical indicating device, which indicates visually the intensity of the respective measured field intensity.

15. Apparatus according to claim 1, characterized by a scriber, practically formed as a felt-tip pencil, which is fixed at the transmitter (6) for an easier and quicker marking of the determined position of the probe (3).

16. Apparatus according to claim 14, characterized by an auxiliary device with which the scriber is movable in direction to the body in order to mark the position respectively the run of the probe.

17. Apparatus according to claim 1, characterized in that the transmitter (6) comprises two devices for producing electromagnetic waves, which are ranged and dimensioned in such a way, that the waves radiated by the two devices form an angle of preferably less than 45° in the measuring plane.

18. Apparatus according to one of the claims 1 to 17, characterized in that the probe consists of two parts (30, 31) fitted into each other concentrically, of which the outer part (30) is formed as a hose.

19. Apparatus according to claim 1, characterized in that the inner part (31) is carried in the outer part (30) in such a way, that it can be pulled out, and the inner part comprises two conductors (4') which extend in the longitudinal direction of the probe and which are connected with the one end to the input of the receiver (5) and with the other end connected together at the front end.

20. Apparatus according to claim 18, characterized in that the inner part (31) comprises two conductors (4'), connected together at the front end, which extend in the longitudinal direction of the probe (3) and which are insulated from each other and twisted with each other in the longitudinal direction.

21. Apparatus according to claim 19 or 20, characterized in that a coil (4A') with at least two turns is provided at the front end of the inner part (31) and that both ends of the coil are arranged on each of the conductors (4') preferably twisted together and provided in the inner part (31).

**Revendications**

1. Dispositif pour déterminer la position respective du bout avant d'une sonde introduite dans un corps vivant, consistant en un poste émetteur mouvant hors du corps, produisant des ondes électromagnétiques, et en un récepteur attaché à la sonde comprenant à l'intérieur un détecteur qui réagit à l'intensité du champ des ondes de l'émetteur tombant sur le récepteur et dont l'indication optique et/ou acoustique est une fonction de l'intensité du champ respective au récepteur, caractérisé par le fait que le récepteur (5) est connecté par une antenne réceptrice en fil de fer qui s'étend de la zone de la sonde (3) hors du corps (2) jusqu'au bout avant (3A) de cette sonde.

2. Dispositif selon la revendication 1, caractérisé par une telle configuration constructive de la sonde (3) réspectivement de l'antenne réceptrice y contenue, que celle-ci a dans une zone une essentiellement plus haute sensibilité pour les ondes électromagnétiques tombant sur elle que dans la zone restante.

3. Dispositif selon la revendication 1, caractérisé par le fait que la sonde (3) a dans sa zone avant une essentiellement plus haute sensibilité pour les ondes électromagnétiques tombant sur elle que dans la zone restante.

4. Dispositif selon la revendication 1 et 2, caractérisé par le fait que dans la zone de la sonde (3) avec une sensibilité plus haute une bobine est placée qui est raccordée à deux conducteurs.

5. Dispositif selon la revendication 1, caractérisé par le fait que la sonde (3) contient deux conducteurs (4) électriques isolés l'un de l'autre et en plus un troisième conducteur électrique dont sa zone du bout avant est connecté avec la zone du bout avant de l'un des deux autres conducteurs.

6. Dispositif selon la revendication 1, caractérisé par le fait que la sonde (3) présente quatre conducteurs distribués essentiellement conforme suivant le périmètre dont chaque deux conducteurs font face diamétralement l'un à l'autre et sont connectés ensemble dans leur zone du bout avant.

7. Dispositif selon la revendication 6, caractérisé par le fait qu'un dispositif de commutation est prévu pour un raccord facultatif du récepteur à un des deux paires de conducteurs connectés ensemble dans leur zone du bout avant.

8. Dispositif selon la revendication 1, caractérisé par le fait que la sonde (3) comporte deux conducteurs (4) formés helicoidalement en sens longitudinal, l'un en face de l'autre diamétralement, et qui sont connectés ensemble dans leur zone de bout avant.

9. Dispositif selon la revendication 1, caractérisé par le fait que la sonde (3) comporte dans sa zone du bout avant une bobine (4A) raccordée aux deux conducteurs (4).

10. Dispositif selon la revendication 1, caractérisé par le fait que la sonde (3) comporte dans sa zone du bout avant une résistance variable avec la température, de préférence une dite thermistance, et/ou un deuxième détecteur physico-chimique, par exemple pour la mesure de la pression partielle de $O_2$, et que les conducteurs d'amenée pour cette résistance variable avec la température respectivement pour le détecteur physico-chimique sont formés en antenne et raccordés au récepteur (5).

11. Dispositif selon la revendication 1, caractérisé par le fait que la fréquence des ondes électromagnétiques produites par le poste émitteur (6) est au nombre entre 15 et 200 kHz.

12. Dispositif selon la revendication 1, caractérisé par le fait que l'intensité et/ou la fréquence des ondes électromagnétiques dégagées est facultatif intercalable réspectivement commutable.

13. Dispositif selon la revendication 1, caractérisé par le fait que le récepteur (5) produit un son audible, dont la fréquence respective et/ou l'intensité du son est une fonction de l'intensité du champ reçu.

14. Dispositif selon la revendication 1, caractérisé par le fait que le récepteur (5) comporte un dispositif indicateur optique, qui montre visuellement l'intensité de l'intensité du champ mesurée à chaque fois.

15. Dispositif selon la revendication 1, caractérisé par un stylo, pratiquement en forme de crayon feutre, placé au poste émitteur (6) en vue d'un marquage plus simple et plus rapide de la position de la sonde (3) déterminée.

16. Dispositif selon la revendication 14, caractérisé par un dispositif auxiliaire avec lequel le stylo est réglable facultatif en direction du corps en vue de marquage de la position réspectivement de l'allure de la sonde.

17. Dispositif selon la revendication 1, caractérisé par le fait que le poste émetteur (6) comporte deux dispositifs pour la production des ondes électromagnétiques, qui sont tellement placés et dimensionnés que les ondes dégagées des deux dispositifs forment sur la surface de mesure ensemble un angle de préférence de moins de 45°.

18. Dispositif selon une des revendications 1–17, caractérisé par le fait que la sonde (3) est composée de deux parties (30, 31) emboîtées concentriquement, dont la partie extérieure est en forme de tuyau flexible.

19. Dispositif selon la revendication 18, caractérisé par le fait que la partie intérieure (31) est montée dans la partie extérieure (30) de façon qu'un puisse la retirer, et qu'elle comporte deux conducteurs s'étendant en sens longitudinal de la sonde, qui sont d'une part connectés avec l'entrée du récepteur (5) et d'autre part connectés ensemble au bout avant.

20. Dispositif selon la revendication 18, caractérisé par le fait que la partie intérieure (31) consiste en deux conducteurs (4') connectés ensemble au bout avant, s'étendant en sens longitudinal de la sonde (3) et qui sont isolés l'un contre l'autre et sont torsadés ensemble en sens longitudinal.

21. Dispositif selon la revendication 19 ou 20, caractérisé par le fait qu'au bout avant de la partie intérieure (31) est prévu une bobine (4A') avec au moins deux spires, dont les deux bouts sont placés à chacun des conducteurs (4') qui se trouvent dans la partie intérieure (31) et qui sont de préférence torsadés réciproquement.

**FIG.1**

**FIG.2**

**FIG.3**

**FIG.4**

FIG. 5

FIG. 6

FIG.7

FIG. 8

FIG.9

FIG. 10

FIG. 11